# EUROPEAN PATENT APPLICATION

(11) **EP 3 162 271 A1**
(43) Date of publication of application: **03.05.2017**
(21) Application number: 15827417.5
(22) Date of filing: 21.07.2015
(51) Int. Cl.: A61B 1/00, A61B 1/04, A61B 1/06, G02B 23/24, G02B 23/26

(54) **ENDOSCOPE SYSTEM**

(30) Priority: 28.07.2014 JP 2014153103
(71) Applicant: Olympus Corporation, Tokyo 192-8507 (JP)
(72) Inventor: HONDA, Kazuki, Hachioji-shi Tokyo 192-8507 (JP); KURA, Yasuhito, Hachioji-shi Tokyo 192-8507 (JP); HINO, Kazuhiko, Hachioji-shi Tokyo 192-8507 (JP)
(74) Representative: Gunzelmann, Rainer
(86) International application number: PCT/JP2015/070725
(87) International publication number: WO 2016/017481

(57) **Abstract**

An endoscope system (1) includes: an insertion portion (6) inserted into a subject; an observation window (8) provided on the insertion portion (6) and configured to acquire a first subject image from a forward direction; an observation window (10) provided on the insertion portion (6) and configured to acquire a second subject image from a lateral direction different from the forward direction; an illumination window (7) configured to emit first illumination light in the forward direction; an illumination window (9) configured to emit second illumination light in the lateral direction; and a control section (42) configured to individually and independently control an amount of light of the first illumination light of the illumination window (7) and an amount of light of the second illumination light of the illumination window (9) based on signals for detecting signals indicating temperatures of the illumination windows (7) and (9).

## Description

### Technical Field

The present invention relates to an endoscope system, and particularly, to an endoscope system configured to radiate illumination light in at least two directions and acquire subject images from the at least two directions.

### Background Art

Conventionally, endoscopes have been widely used in a medical field and an industrial field. An endoscope includes illumination means and observation means on a distal end side of an insertion portion, and the endoscope can be inserted into a subject to observe and inspect inside of the subject.

In recent years, an endoscope that can observe two or more directions is proposed, and for example, an endoscope is proposed as disclosed in Japanese Patent No. 4782900, the endoscope including a forward field of view in which a front side of an insertion portion is an observation field of view and including a lateral field of view in which a side surface of the insertion portion is an observation field of view. By using the endoscope, an inspector can observe two directions, forward and lateral directions, at the same time.

However, to obtain bright images in the fields of view in the respective directions by using the endoscope that can observe two or more directions, illumination corresponding to the fields of view in the respective directions is necessary. Therefore, an amount of heat generation of a distal end portion of the insertion portion increases with an increase in illumination portions, and the distal end portion may overheat.

Accordingly, an object of the present invention is to provide an endoscope system configured to prevent overheating of a distal end portion in an endoscope that can observe two or more directions.

### Disclosure of Invention

### Means for Solving the Problem

An aspect of the present invention provides an endoscope system including: an insertion portion inserted into a subject; a subject image acquisition portion provided on the insertion portion and configured to acquire an image of the subject; a first illumination portion provided on the insertion portion and configured to emit first illumination light to a first region of the subject; a second illumination portion provided on the insertion portion and configured to emit second illumination light to a second region of the subject at least partially different from the first region; a signal detection section configured to detect a first signal indicating a temperature of the first illumination portion and a second signal indicating a temperature of the second illumination portion; and an illumination light amount control section configured to individually and independently control an amount of light of the first illumination light and an amount of light of the second illumination light based on the first signal and the second signal.

### Brief Description of the Drawings

Fig. 1 is a configuration diagram showing a configuration of an endoscope system according to a first embodiment of the present invention;
Fig. 2 is a cross-sectional view of a distal end portion 6a of an insertion portion 6 according to the first embodiment of the present invention;
Fig. 3 is a diagram showing an example of a display screen of an endoscopic image displayed on a display apparatus 5 according to the first embodiment of the present invention;
Fig. 4 is a flowchart showing an example of a flow of control action of two apertures 31a and 31b by a control section 42 according to the first embodiment of the present invention;
Fig. 5 is a configuration diagram showing a configuration of an endoscope system 1A according to a second embodiment of the present invention;
Fig. 6 is a diagram for describing configurations of an aperture 31c and a light shielding plate 37 of a light source apparatus 3 according to the second embodiment of the present invention;
Fig. 7 is a flowchart showing an example of a flow of control action of the light shielding plate 37 by a control section 42A according to the second embodiment of the present invention;
Fig. 8 is a configuration diagram showing a configuration of an endoscope system 1B according to a third embodiment of the present invention;
Fig. 9 is a flowchart showing an example of a flow of control action of a brightness target value of an endoscopic image by a control section 42B according to the third embodiment of the present invention;
Fig. 10 is a graph showing a change in an amount of opening of the aperture 31c with a lapse of time period according to the third embodiment of the present invention;
Fig. 11 is a configuration diagram showing a configuration of an endoscope system 1C according to a fourth embodiment of the present invention;
Fig. 12 is a diagram showing an example of the display screen of endoscopic images displayed on the display apparatus 5 according to the fourth embodiment of the present invention;
Fig. 13 is a diagram for describing drive control of six light-emitting devices respectively located on six illumination windows 7a, 7b, 9a, 9b, 9c, and 9d located on the distal end portion 6a according to the fourth embodiment of the present invention;
Fig. 14 is a diagram for describing drive control of six light-emitting devices respectively located on the six illumination windows 7a, 7b, 9a, 9b, 9c, and 9d located on the distal end portion 6a according to a fifth embodiment of the present invention;
Fig. 15 is a graph showing a change in a drive signal level for the light-emitting devices with a lapse of time period according to the fifth embodiment of the present invention;
Fig. 16 is a diagram showing a display system using three display apparatuses 5; and
Fig. 17 is a perspective view of the distal end portion 6a of the insertion portion 6 provided with a unit for lateral observation.

### Best Mode for Carrying Out the Invention

Hereinafter, embodiments of the present invention will be described with reference to the drawings.

Note that scaling of each constituent element varies in each drawing used in the following description in order to illustrate each constituent element in a size that allows recognizing the constituent element on the drawing, and the present invention is not limited only to quantities of the constituent elements, shapes of the constituent elements, ratios of the sizes of the constituent elements, and relative positional relationships between respective constituent elements described in the drawings.

### (First Embodiment)

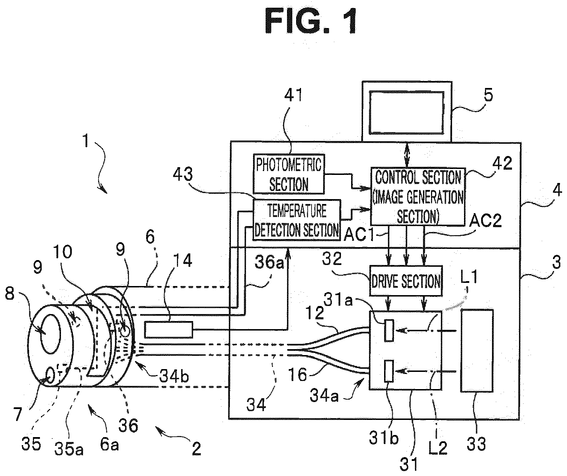

Fig. 1 is a configuration diagram showing a configuration of an endoscope system according to the present embodiment. An endoscope system 1 includes an endoscope 2, a light source apparatus 3, a processor 4, and a display apparatus 5.

The endoscope 2 includes: an insertion portion 6 inserted into a subject; and an operation portion not shown. The endoscope 2 is connected to the light source apparatus 3 and the processor 4 through a cable not shown. A distal end portion 6a of the insertion portion 6 of the endoscope 2 is provided with: an illumination window 7 and an observation window 8 for forward field of view; and two illumination windows 9 and an observation window 10 for lateral field of view.

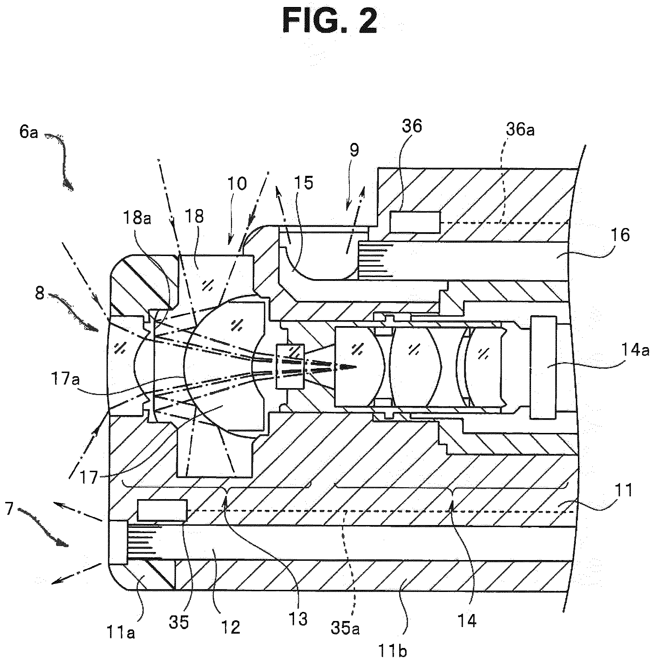

Fig. 2 is a cross-sectional view of the distal end portion 6a of the insertion portion 6. Note that only one illumination window 9 for lateral observation is illustrated in Fig. 2.

The distal end portion 6a of the insertion portion 6 includes a distal end rigid member 11, and the illumination window 7 is provided on a distal end surface of the distal end rigid member 11. A distal end surface of a forward illumination light guide 12 is located on a back side of the illumination window 7. The observation window 8 is provided on the distal end surface of the distal end rigid member 11. An objective optical system 13 is located on a back side of the observation window 8. An image pickup unit 14 is located on a back side of the objective optical system 13. Note that a cover 11a is attached to a distal end portion of the distal end rigid member 11. The insertion portion 6 is covered with an outer skin 11b.

That is, forward illumination light is emitted from the illumination window 7, and reflected light from an observation site in the subject enters the observation window 8.

The two illumination windows 9 are located on a side surface of the distal end rigid member 11, and a distal end surface of a lateral illumination light guide 16 is located behind each illumination window 9 through a mirror 15 in which a reflecting surface is a curved surface.

Therefore, the illumination window 7 configures a first illumination portion that emits first illumination light to a region including a forward direction as a first region inside of the subject. The plurality of illumination windows 9 configure a second illumination portion that emits second illumination light to a region including a lateral direction as a second region at least partially different from the first direction.

The second region different from the first region denotes that optical axes in the respective regions are in different directions. A subject image in the first region and a subject image in the second region may partially overlap or may not overlap. An irradiation range of the first illumination light and an irradiation range of the second illumination light may partially overlap or may not overlap.

The observation window 10 is located on the side surface of the distal end rigid member 11, and the objective optical system 13 is located on a back side of the observation window 10. The objective optical system 13 is configured to direct reflected light from the forward direction passing through the observation window 8 and reflected light from the lateral direction passing through the observation window 10 toward the image pickup unit 14. In Fig. 2, the objective optical system 13 includes two optical members 17 and 18. The optical member 17 is a lens including a convex surface 17a, and the optical member 18 includes a reflecting surface 18a for reflecting reflected light from the lateral direction toward the image pickup unit 14 through the optical member 17.

That is, the observation window 8 is provided on the insertion portion 6 and configures a first subject image acquisition portion that acquires an image from the forward direction included in the first region. The observation window 10 is provided on the insertion portion 6 and configures a second subject image acquisition portion that acquires an image from the lateral direction included in the second region different from the forward direction. The observation window 10 is arranged closer to a proximal end side of the insertion portion 6 relative to the observation window 8.

More specifically, the image from the forward direction included in the first region is a subject image of the first region including the forward direction of the insertion portion 6 substantially parallel to a longitudinal direction of the insertion portion 6. The image from the lateral direction included in the second region is a subject image of the second region including the lateral direction of the insertion portion 6 in a direction intersecting (for example, substantially orthogonal to) the longitudinal direction of the insertion portion 6. The observation window 8 is a forward subject acquisition portion configured to acquire the subject image of the first region including the forward direction of the insertion portion 6, and the observation window 10 is a lateral subject image acquisition portion configured to acquire the subject image of the second region including the lateral direction of the insertion portion 6.

The observation window 8 as a subject image acquisition portion is arranged on the distal end portion 6a of the insertion portion 6 in a direction in which the insertion portion 6 is inserted. The observation window 10 as a subject image acquisition portion is arranged on a side surface portion of the insertion portion 6, in an outer diameter direction of the insertion portion 6. The image pickup unit 14 as an image pickup section is arranged to photoelectrically convert the subject image from the observation window 8 and the subject image from the observation window 10 on the same image pickup surface and is electrically connected to the processor 4 as an image processing section.

That is, the observation window 8 is arranged on the distal end portion 6a in the longitudinal direction of the insertion portion 6 so as to acquire a first subject image from a first direction that is a direction in which the insertion portion 6 is inserted. The observation window 10 is arranged in a circumferential direction of the insertion portion 6 so as to acquire a second subject image from a second direction different from the first direction.

Therefore, the forward illumination light is emitted from the illumination window 7, and the reflected light from the subject enters the image pickup unit 14 through the observation window 8. The lateral illumination light is emitted from the two illumination windows 9, and the reflected light from the subject enters the image pickup unit 14 through the observation window 10. An image pickup device 14a of the image pickup unit 14 photoelectrically converts an optical image of the subject and outputs an image pickup signal to the processor 4.

Returning to Fig. 1, the image pickup signal from the image pickup unit 14 is supplied to the processor 4 as an image processing section, and an image processing circuit not shown generates an endoscopic image. The processor 4 outputs image data of the endoscopic image to the display apparatus 5. The processor 4 is an image generation section, and the display apparatus 5 is a display section configured to display the image generated by the processor 4.

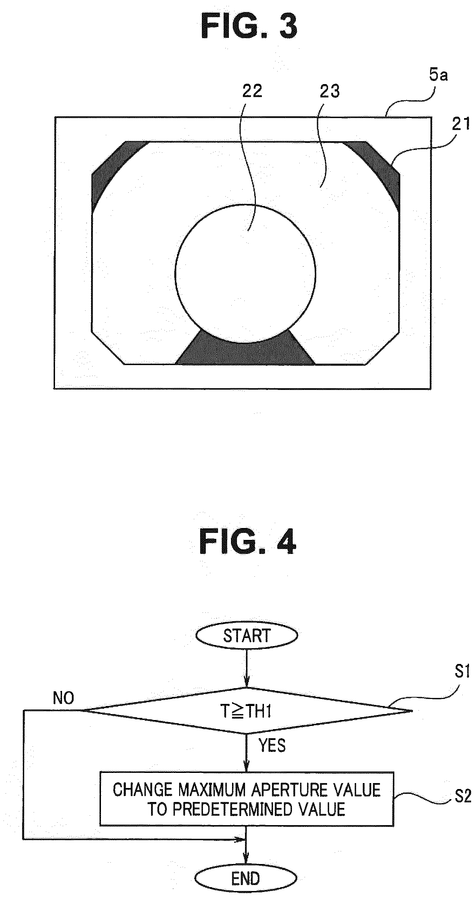

Fig. 3 is a diagram showing an example of a display screen of the endoscopic image displayed on the display apparatus 5.

An endoscopic image 21 displayed on a display screen 5a of the display apparatus 5 is a substantially rectangular image and includes two regions 22 and 23. A circular region 22 at a center part is a region for displaying a forward observation image, and a C-shaped region 23 around the region 22 at the center part is a region for displaying a lateral observation image.

That is, the forward observation image is displayed on the display screen 5a of the display apparatus 5 in a substantially circular shape, and the lateral observation image is displayed on the display screen 5a in a substantially annular shape surrounding at least part of the surroundings of the forward observation image (adjacent to the forward observation image). Therefore, a wide-angle endoscopic image is displayed on the display apparatus 5.

Returning to Fig. 1, the light source apparatus 3 includes: a light-adjusting section 31; a drive section 32 configured to drive the light-adjusting section 31; and a light source 33.

A light guide 34 includes the forward illumination light guide 12 and the lateral illumination light guide 16. A distal end portion of the lateral illumination light guide 16 is branched into two parts. The forward illumination light guide 12 and the lateral illumination light guide 16 are independent from each other. The forward illumination light guide 12 transmits light to the illumination window 7, and the lateral illumination light guide 16 transmits light to the two illumination windows 9.

The light source 33 includes a lamp, such as a xenon lamp, configured to emit white light. The light from the light source 33 enters the light guide 34 through the light-adjusting section 31 and is emitted from a distal end portion 34b of the light guide 34.

A light condensing apparatus not shown condenses the light emitted from the light-adjusting section 31 on respective proximal end surfaces of the forward illumination light guide 12 and the lateral illumination light guide 16 of a proximal end portion 34a of the light guide 34, and the light enters the light guide 34.

The light entering the proximal end surface of the forward illumination light guide 12 is emitted from the illumination window 7 through a distal end surface of the forward illumination light guide 12. The light entering the proximal end surface of the lateral illumination light guide 16 is emitted from each of the illumination windows 9 through respective end surfaces of the two branched distal end portions of the lateral illumination light guide 16.

The light-adjusting section 31 adjusts an amount of light of the light from the light source 33. More specifically, the light-adjusting section 31 includes two apertures 31a and 31b. The aperture 31a adjusts an amount of light of light L1 for forward illumination based on an aperture control signal AC1 from a control section 42. The aperture 31b adjusts an amount of light of light L2 for lateral illumination based on an aperture control signal AC2 from the control section 42.

The apertures 31a and 31b may be any apertures, such as apertures using fan-shaped mask members and apertures in which an amount of opening of an opening portion at the center changes according to motion of a plurality of aperture blades. The apertures 31a and 31b are driven by a drive mechanism such as a motor.

The processor 4 includes a photometric section 41, the control section 42, and a temperature detection section 43.

The photometric section 41 is a processing section configured to calculate brightness of each of the two regions 22 and 23 of the endoscopic image 21 described above from the image data of the endoscopic image generated in the processor 4. The photometric section 41 calculates the brightness of the region 22 and the brightness of the region 23 and outputs them to the control section 42. The brightness of each region is an average value of luminance of all pixels in each region.

A temperature sensor 35 is provided near the illumination window 7 in the distal end portion 6a. A temperature sensor 36 is further provided near one of the two illumination windows 9.

Output signals of the temperature sensors 35 and 36 are inputted to the temperature detection section 43 through signal lines 35a and 36a, respectively. The temperature detection section 43 outputs temperature data of the illumination window 7 to the control section 42 based on the output signal of the temperature sensor 35. Similarly, the temperature detection section 43 outputs temperature data of the illumination window 9 to the control section 42 based on the output signal of the temperature sensor 36. Therefore, the control section 42 can monitor the temperature data of the respective illumination portions 7 and 9 all the time. That is, the temperature detection section 43 configures a signal detection section that detects signals indicating temperatures of the illumination window 7 and the illumination window 9 from the temperature sensor 35 and the temperature sensor 36. More specifically, the signals indicating the temperatures include a first signal that is an output signal of the temperature sensor 35 provided near the illumination window 7 for forward field of view and a second signal that is an output signal of the temperature sensor 36 provided near the illumination window 9 for lateral field of view.

Note that two temperature sensors 36 may be provided on the two illumination windows 9. The temperature detection section 43 may obtain an average value or the like of the temperatures of the two illumination windows 9 for lateral field of view from output signals of the two temperature sensors, and data of the average value or the like may be outputted to the control section 42.

The control section 42 generates the aperture control signals AC 1 and AC2 for individually and independently controlling the two apertures 31a and 31b, respectively, based on the brightness of each of the two regions 22 and 23 of the endoscopic image 21 detected by the photometric section 41 and outputs the aperture control signals AC1 and AC2 to the drive section 32.

The drive section 32 individually and independently controls the amount of opening of each of the apertures 31a and 31b based on the aperture control signals AC1 and AC2 from the control section 42, respectively.

The control section 42 can set, for the drive section 32, a maximum aperture value of each of the apertures 31a and 31b. The drive section 32 drives each of the apertures 31 a and 31b in a range not exceeding the set maximum aperture value.

### (Action)

Fig. 4 is a flowchart showing an example of a flow of control action of the two apertures 31a and 31b by the control section 42. A process of Fig. 4 is executed for each of the apertures 31a and 31b based on the output signal of each temperature sensor. Therefore, the control section 42 configures an illumination light amount control section that individually and independently controls the amount of light of at least one of the illumination light of the illumination window 7 and the illumination light of the illumination window 9 based on the output signal of each temperature sensor. More specifically, the control section 42 limits the maximum aperture value of the apertures 31a and 31b for limiting the amount of light of the illumination light for forward field of view and for lateral field of view to thereby control the amount of light of at least one of the illumination light for forward field of view and the illumination light for lateral field of view. The amount of light of at least one of the illumination light of the illumination window 7 and the illumination light of the illumination window 9 can be individually and independently controlled to independently increase or decrease only the amount of light of necessary part of the illumination light of the illumination window 7 and the illumination light of the illumination window 9 to prevent the entire endoscopic image 21 displayed on the display screen 5a of the display apparatus 5 from becoming dark.

The control section 42 judges whether temperature data T of each of the illumination windows 7 and 9 from the temperature detection section 43 is equal to or greater than a predetermined value TH1 (S1). The predetermined value TH1 is, for example, 37°C.

If it is judged that the temperature data T of the illumination window 7 or the illumination window 9 is equal to or greater than the predetermined value TH1 (S1: YES), the control section 42 changes, to a predetermined value AD, the setting of a maximum aperture value DM of the apertures 31a and 31b for controlling the amount of emitted light of the illumination window 7 or the illumination window 9 in which it is judged that the temperature data T is equal to or greater than the predetermined value TH1.

For example, it is assumed that the apertures 31a and 31b can be controlled in a range of 0 to 100, the maximum aperture value DM is set to 100 in the drive section 32, and the drive section 32 controls each of the apertures 31a and 31b under the control by the control section 42. When the temperature data T of one of the illumination windows 9 for lateral field of view becomes equal to or greater than the predetermined value TH1 during the endoscopic observation, the control section 42 changes, for the drive section 32, the maximum aperture value DM of the aperture 31b to 75 that is the predetermined value AD. As a result, the drive section 32 controls the amount of opening of the aperture 31b based on the aperture control signal AC2 from the control section 42 to prevent the amount of opening from exceeding the maximum aperture value DM. Therefore, a rise in temperature of the illumination window 9 for lateral field of view is suppressed.

Note that when the temperature data T of the illumination window 7 or 9 becomes smaller than the predetermined value TH1 after the change in the maximum aperture value DM, the maximum aperture value DM is changed to the original value such as 100.

That is, a rise in the temperature of the illumination window in which the temperature has risen is suppressed, and overheating of the distal end portion 6a of the insertion portion 6 is prevented. Furthermore, the amount of emitted light of the illumination window with the temperature data T smaller than the predetermined value TH1 does not decrease, and the image obtained by the observation window corresponding to the illumination window with the temperature data T smaller than the predetermined value TH1 becomes a clear image.

Therefore, according to the present embodiment, the temperatures of two or more illumination windows with different illumination regions are individually checked, and the two or more illumination windows are individually and independently controlled to prevent the temperatures from rising above a predetermined temperature in an endoscope that can observe two or more directions. This can provide an endoscope system that can perform detailed illumination control in which the amounts of light of all illuminations do not change at the same time and that can prevent overheating of the distal end portion.

### (Second Embodiment)

A second embodiment relates to an endoscope system that prioritizes the forward field of view over the lateral field of view to limit the illumination for lateral field of view to prevent the temperature of the distal end portion of the insertion portion from becoming high.

An endoscope system 1A of the present embodiment has substantially the same configuration as the endoscope system 1 of the first embodiment. Therefore, in the present embodiment, the same reference signs are provided to the same constituent elements as in the endoscope system 1 of the first embodiment, and the description will not be repeated.

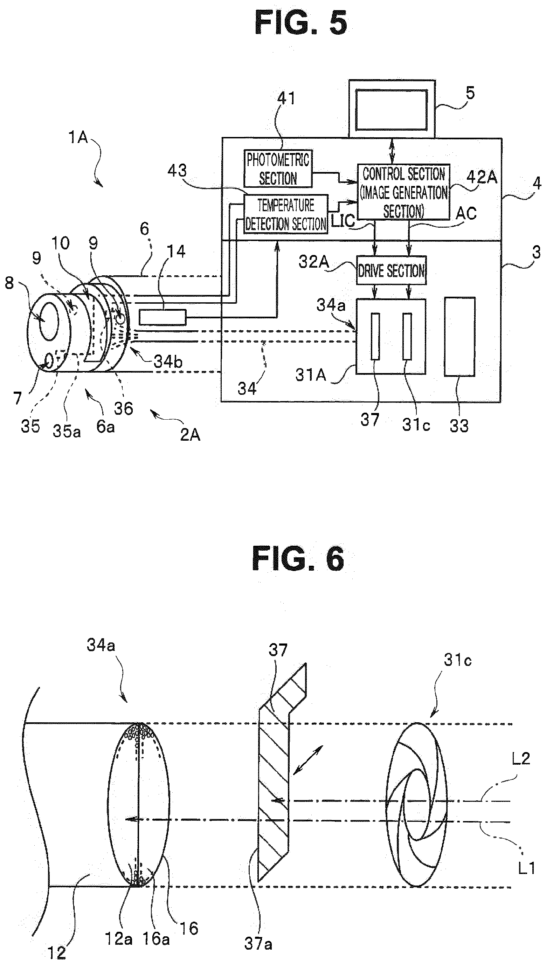

Fig. 5 is a configuration diagram showing the configuration of the endoscope system 1A according to the present embodiment.

The distal end portion 6a of the insertion portion 6 of an endoscope 2A has substantially the same configuration as the distal end portion of the first embodiment.

A light-adjusting section 31A of the light source apparatus 3 includes an aperture 31c and a light shielding plate 37.

Fig. 6 is a diagram for describing a configuration of the aperture 31 c and the light shielding plate 37 of the light source apparatus 3. The aperture 31c has a structure in which an amount of opening of an opening portion at the center changes according to motion of a plurality of aperture blades to adjust the amount of light passing from the light source 33.

The light shielding plate 37 is a plate-like member that does not transmit light. The light shielding plate 37 can be moved by an actuator not shown to shield part of the light from the aperture 31c between the aperture 31 c and the proximal end portion 34a of the light guide 34.

When the light shielding plate 37 is positioned so as not to shield part of the light from the aperture 31c, that is, when the light shielding plate 37 is drawn back, the light shielding plate 3 does not shield part of the light from the aperture 31 c. However, when the light shielding plate 37 is positioned so as to shield part of the light from the aperture 31c, that is, when the light shielding plate 37 is protruding, the light shielding plate 37 shields part of the light from the aperture 31 c.

The proximal end portion 34a of the light guide 34 is divided into a proximal end surface region 12a of the forward illumination light guide 12 and a proximal end surface region 16a of the lateral illumination light guide 16. As shown in Fig. 6, one of two semicircular regions of an end surface of the circular proximal end portion 34a is the proximal end surface region 12a, and the other of the two semicircular regions is the proximal end surface region 16a.

When the light shielding plate 37 is protruding, the light shielding plate 37 moves to between the aperture 31 c and the proximal end portion 34a to prevent the light passing through the aperture 31c from entering the proximal end surface region 16a of the lateral illumination light guide 16.

Therefore, the shape of the light shielding plate 37 is formed such that the light shielding plate 37 prevents the light passing through the aperture 31 c from entering the proximal end surface region 16a of the lateral illumination light guide 16 when the light shielding plate 37 is protruding.

In the case of Fig. 6, the light shielding plate 37 includes a linear end portion 37a to precisely prevent the light L2 for lateral illumination from the aperture 31c from entering the proximal end surface region 16a of the lateral illumination light guide 16 when the light shielding plate 37 is protruding. When the light shielding plate 37 is protruding, the light L1 for forward illumination is not shielded.

A control section 42A generates an aperture control signal AC for controlling the aperture 31c based on the brightness of each of the two regions 22 and 23 of the endoscopic image 21 detected by the photometric section 41 and outputs the aperture control signal AC to a drive section 32A. The drive section 32A controls the amount of opening of the aperture 31 c based on the aperture control signal AC from the control section 42.

The control section 42A further generates a light shielding plate drive signal LIC for driving the light shielding plate 37 based on the aperture control signal AC and outputs the light shielding plate drive signal LIC to the drive section 32A. The drive section 32A controls the position of the light shielding plate 37 based on the light shielding plate drive signal LIC from the control section 42.

### (Action)

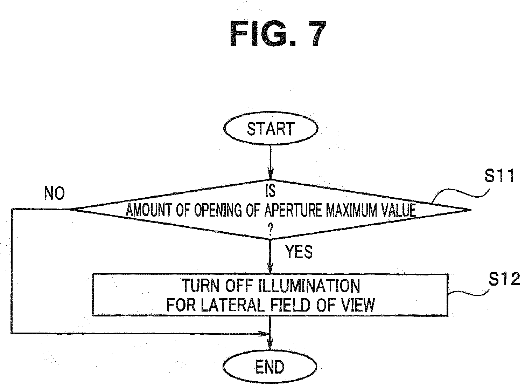

Fig. 7 is a flowchart showing an example of a flow of control action of the light shielding plate 37 of the control section 42A. As described, the control section 42A controls the amount of opening of the aperture 31 c based on the brightness of the endoscopic image. The control section 42A also executes a process of Fig. 7 while controlling the aperture 31 c.

The control section 42A judges whether the amount of opening of the aperture 31c is a maximum value DM1 (S11). More specifically, whether the aperture control signal AC is the maximum value DM1 is judged. For example, when the aperture is controlled in a range of 0 to 100, whether the aperture control signal AC is 100 is judged.

Here, the maximum value DM1 is the amount of opening of the aperture 31c when it is determined that the predetermined site of the distal end portion 6a is equal to or higher than a predetermined temperature, such as 37°C, as in the method of the first embodiment.

Therefore, in other words, the control section 42A that executes the process of S11 configures a signal detection section that detects signals indicating the temperatures of the illumination portions of the illumination windows 7 and 9. More specifically, the signals indicating the temperatures are signals of the amount of opening of the aperture 31c for limiting the amount of the light of the illumination light for forward field of view and lateral field of view.

When the amount of opening of the aperture 31c is the maximum value DM1 (S11), that is, when it is estimated that the distal end portion 6a is equal to or higher than the predetermined temperature, the control section 42A turns off the illumination for lateral field of view through the light shielding plate 37 (S12). More specifically, the control section 42A outputs the light shielding plate control signal LIC and drives the light shielding plate 37 to prevent the light from the aperture 31 c from entering the proximal end surface region 16a of the lateral illumination light guide 16.

That is, the control section 42 that executes the process of S12 configures an illumination light amount control section that controls the amount of light of at least one of the illumination light of the illumination window 7 and the illumination light of the illumination window 9 based on the signals indicating the temperatures of the illumination portions of the illumination windows 7 and 9. More specifically, the control section 42 limits the amount of light of the illumination light for lateral field of view that is the at least one of the amounts of light.

There is no light entering the lateral illumination light guide 16 due to the light shielding plate 37. Therefore, a rise in the temperature of the illumination window 9 for lateral field of view is suppressed, and as a result, a rise in the temperature of the distal end portion 6a is also suppressed.

Note that when the amount of opening of the aperture is not the maximum value DM1 any more after the light shielding plate 37 is moved to prevent the light from entering the lateral illumination light guide 16, the control section 42A moves the light shielding plate 37 to cause the light from the aperture 31c to enter the proximal end surface region 16a of the lateral illumination light guide 16.

If the amount of opening of the aperture 31c is not the maximum value DM1 (S11: NO), the process does not do anything.

As described, the control section 42A turns off the illumination for lateral field of view when it is estimated that the temperature of the distal end portion 6a is equal to or higher than the predetermined temperature, and a rise in the temperature of the distal end portion 6a can be suppressed.

In the case of the endoscope, the illumination for forward field of view is not turned off even when the illumination for lateral field of view is turned off, and the forward field of view is ensured. Therefore, the surgeon can insert or remove the insertion portion 6.

Note that although the light shielding plate 37 is used to suppress the entrance of the light into the lateral illumination light guide 16 in the example described above, a neutral density filter may also be used to reduce the amount of entering light. Although the illumination for lateral field of view is turned off based on whether the amount of opening of the aperture 31c is the maximum value DM1, the illumination amount of the illumination for lateral field of view may be reduced in stages according to the amount of opening of the aperture 31 c.

For example, when the amount of opening of the aperture 31c is between 80 and 90, the amount of light of the illumination for lateral field of view may be reduced to 50% of the maximum amount of light. When the amount of opening of the aperture 31 c is between 90 and 100, the amount of light of the illumination for lateral field of view may be reduced to 25%. When the amount of opening of the aperture 3c becomes 100, the amount of light of the illumination for lateral field of view may be reduced to 0%.

In this case, when the amount of opening of the aperture 31c becomes between 90 and 100 after the amount of light of the illumination for lateral field of view is once set to 0%, the amount of light of the illumination for lateral field of view is increased to 25% of the maximum amount of light. When the amount of opening of the aperture 31c becomes between 80 and 90, the amount of light of the illumination for lateral field of view is increased to 50%. When the amount of opening of the aperture 31 c becomes less than 80, the amount of light of the illumination for lateral field of view is increased to 100%. The action of the light shielding plate 37 is controlled in this way.

Therefore, according to the present embodiment, the temperatures of two or more illumination windows with different illumination regions are individually checked in an endoscope that can observe two or more directions. The amounts of light for the two or more illumination windows are individually and independently controlled, and the value of the aperture is adjusted at the same time to prevent the temperatures from rising above a predetermined temperature. This can provide an endoscope system that can perform detailed illumination control in which the amounts of light of all illuminations do not change at the same time and that can prevent overheating of the distal end portion.

### (Third Embodiment)

The endoscope system of the second embodiment prioritizes the forward field of view over the lateral field of view, estimates the temperature of the distal end portion 6a based on the amount of opening of the aperture, and limits the illumination of the lateral field of view to prevent the temperature of the distal end portion of the insertion portion from becoming high. An endoscope system of the present embodiment relates to an endoscope system that estimates the temperature of the distal end portion 6a based on the change over time of the amount of opening of the aperture to control the illumination to prevent the temperature of the distal end portion of the insertion portion from becoming high.

An endoscope system 1B of the present embodiment has substantially the same configuration as the endoscope system 1A of the second embodiment. In the present embodiment, the same reference signs are provided to the same constituent elements as in the endoscope system 1A of the second embodiment, and the description will not be repeated.

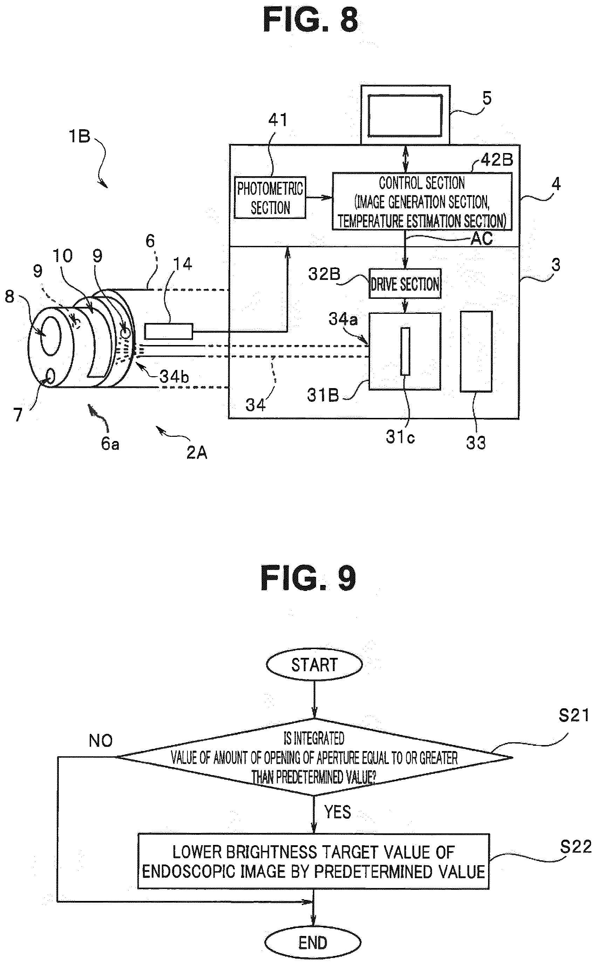

Fig. 8 is a configuration diagram showing a configuration of the endoscope system 1B according to the present embodiment.

The distal end portion 6a of the insertion portion 6 has the same configuration as the distal end portion of the first embodiment, except that the temperature sensor of the first embodiment is not provided. A light-adjusting section 31B of the light source apparatus 3 includes the aperture 31 c.

A control section 42B generates the aperture control signal AC for controlling the aperture 31c based on the brightness of each of the two regions 22 and 23 of the endoscopic image 21 detected by the photometric section 41 and outputs the aperture control signal AC to a drive section 32B. The drive section 32B controls the amount of opening of the aperture 31 c based on the aperture control signal AC from the control section 42B.

### (Action)

Fig. 9 is a flowchart showing an example of a flow of control action of a brightness target value of the endoscopic image of the control section 42B. As described, the control section 42B controls the amount of opening of the aperture 31 c based on the brightness of the endoscopic image. The control section 42B also executes a process of Fig. 9 while controlling the aperture 31 c.

The control section 42B judges whether an integrated value of the amount of opening of the aperture 31c in a past predetermined period PT is equal to or greater than a predetermined value TH2 (S21). The control section 42B that executes the process of S21 configures a signal detection section that detects a signal indicating the temperatures of the illumination portions of the illumination windows 7 and 9. More specifically, the signal indicating the temperatures is a signal of the integrated value of the amount of opening of the aperture 31c for limiting the amount of light of the illumination light of the illumination windows 7 and 9 in a predetermined time period.

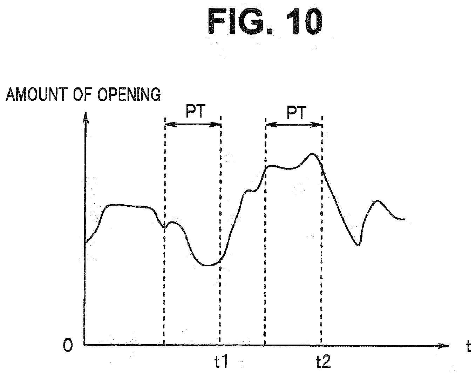

Fig. 10 is a graph indicating a change in the amount of opening of the aperture 31c with a lapse of time period. As shown in Fig. 10, the amount of opening of the aperture 31c changes as indicated by a solid line. As described, the amount of opening of the aperture 31c is controlled by the control section 42B and changes based on the brightness of each of the two regions 22 and 23 of the endoscopic image 21 detected by the photometric section 41.

Comparing time t1 and time t2 in Fig. 10 for example, the amount of opening of the aperture 31c in the most recent past predetermined period PT at the time t2 is greater than the amount of opening of the aperture 31c in the most recent past predetermined period PT at the time t1. Therefore, as for the integrated value of the amount of opening in the past predetermined period PT, the integrated value at the time t2 is also greater than the integrated value at the time t1.

When the integrated value of the most recent past amount of opening is large, there is a possibility that the temperature of the distal end portion 6a is rising. That is, if the integrated value of the amount of opening of the aperture 31c in the most recent predetermined period PT is equal to or greater than the predetermined value TH2, the control section 42B as a temperature estimation section estimates that the temperature of the distal end portion 6a is about to exceed a predetermined temperature, such as 37 degrees, or the temperature is already exceeding the predetermined temperature.

Therefore, the control section 42B as a signal detection section detects the signal indicating the temperature of the illumination portion of the illumination window 7 and the signal indicating the temperature of the illumination portion of the illumination window 9 and lowers the brightness target value of the endoscopic image by a predetermined value BL (S22). The brightness target value of the endoscopic image is a target value of the brightness of the endoscopic image obtained by the image pickup unit 14, and when the brightness target value is lowered by the predetermined value BL, the control section 42B outputs the aperture control signal AC in which the amount of opening of the aperture 31 c is reduced by the predetermined value. That is, the control section 42 that executes the process of S22 configures an illumination light amount control section that controls the amount of light of at least one of the illumination light of the illumination window 7 and the illumination light of the illumination window 9 based on the signals indicating the temperatures of the illumination portions of the illumination windows 7 and 9. More specifically, the control section 42B lowers the target value of the brightness of the subject image of the forward field of view and the subject image of the lateral field of view to control at least one of the amounts of light.

As a result, the amount of light supplied to the distal end portion 6a is reduced, and a rise in the temperature of the distal end portion 6a can be suppressed.

If the integrated value of the amount of opening of the aperture 31c in the most recent predetermined period PT is not equal to or greater than the predetermined value TH2 (S21: NO), the process does not do anything.

In this way, when it is determined that the temperature of the distal end portion 6a is equal to or higher than the predetermined temperature, the control section 42B lowers the brightness target value of the image by the predetermined value BL, and a rise in the temperature of the distal end portion 6a can be suppressed.

Note that although the brightness target value of the image is lowered based on whether the integrated value of the amount of opening of the aperture 31c in the most recent predetermined period PT is equal to or greater than the predetermined value TH2 in the example described above, the brightness target value of the image may be reduced in stages according to the integrated value of the amount of opening of the aperture 31c.

For example, when the integrated value of the amount of opening of the aperture 31c is between AC1 and AC2, the brightness target value of the image may be reduced by 10%. When the integrated value of the amount of opening of the aperture 31c is between AC2 and AC3, the brightness target value of the image may be reduced by 20%. When the integrated value of the amount of opening of the aperture 31c is equal to or greater than AC3, the brightness target value of the image may be reduced by 30%.

In this case, when the integrated value of the amount of opening of the aperture 31 c is reduced subsequently, and the integrated value of the amount of opening of the aperture 31c becomes between AC2 and AC3, the brightness target value of the image is increased to the level of 20% reduction. When the integrated value of the amount of opening of the aperture 31c becomes between AC1 and AC2, the brightness target value of the image is increased to the level of 10% reduction. When the brightness target value of the image becomes less than AC1, the brightness target value of the image is not reduced.

Note that when the brightness target value of the image is reduced by the predetermined value BL or in stages, and the brightness of the image becomes equal to or smaller than predetermined brightness BR1, the brightness of the image may be adjusted by gain adjustment.

Therefore, according to the present embodiment, the temperatures of two or more illumination windows with different illumination regions are individually estimated, and the amounts of light for the two or more illumination windows are individually and independently controlled to prevent the temperatures from rising above a predetermined temperature in an endoscope that can observe two or more directions. This can provide an endoscope system that can perform detailed illumination control in which the amounts of light of all illuminations do not change at the same time and that can prevent overheating of the distal end portion.

### (Fourth Embodiment)

Although one image pickup device receives subject images of both of the forward field of view and the lateral field of view in the endoscope systems of the first, second, and third embodiments, three image pickup devices are used in an endoscope system of the present embodiment, and one image pickup device is configured to receive a subject image of the forward field of view. Two image pickup devices are configured to receive two subject images of the lateral field of view.

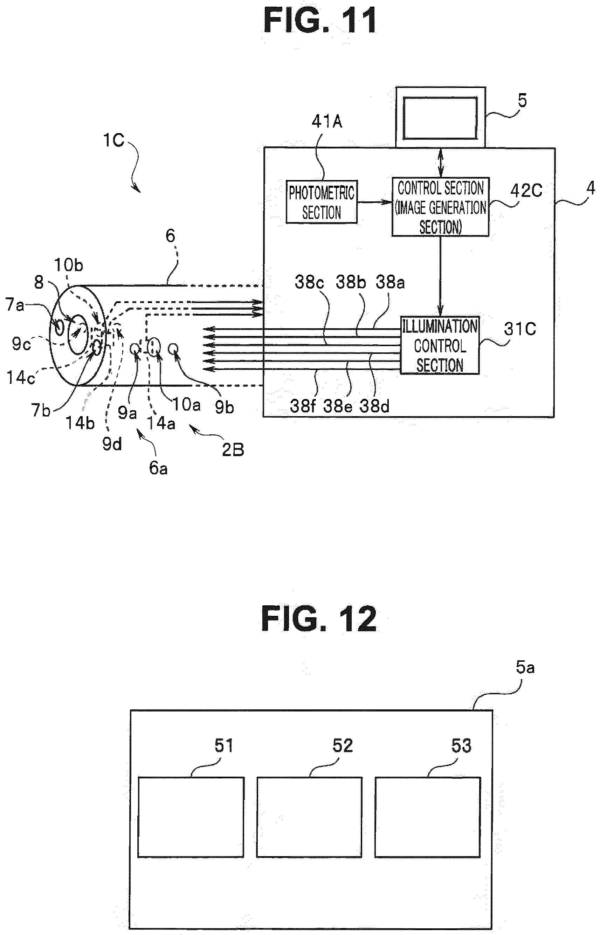

Fig. 11 is a configuration diagram showing a configuration of an endoscope system 1C according to the present embodiment. The endoscope system 1C of the present embodiment has substantially the same configuration as the endoscope system 1B of the third embodiment. Therefore, the same reference signs are provided to the same constituent elements as in the endoscope system 1B, and the description will not be repeated. Different components will be described.

As shown in Fig. 11, an endoscope 2B includes a plurality of illumination windows, such as six illumination windows. Two illumination windows 7a and 7b are for the forward illumination, and four illumination windows 9a, 9b, 9c, and 9d are for the lateral illumination.

The illumination windows 7a and 7b configure a first illumination portion that emits first illumination light to a region including the forward direction of the insertion portion 6 as a first region inside of the subject. The plurality of illumination windows 9a and 9b and illumination windows 9c and 9d configure a second illumination portion that emits second illumination light to a region including the lateral direction of the insertion portion 6 as a second region at least partially different from the first direction.

The second region different from the first region denotes that optical axes in the respective regions are in different directions. A subject image in the first region and a subject image in the second region may partially overlap or may not overlap. An irradiation range of the first illumination light and an irradiation range of the second illumination light may partially overlap or may not overlap.

The endoscope 2B further includes three observation windows. One observation window 8 is for the forward field of view, and two observation windows 10a and 10b are for the lateral field of view.

As shown in Fig. 11, the observation window 8 is arranged on the distal end surface of the distal end portion 6a, and two illumination windows 7a and 7b are located near the observation window 8.

Two observation windows 10a and 10b for observing lateral directions that are directions opposite to each other (for example, left and right directions of the distal end portion 6a) are arranged on a side surface of the distal end portion 6a. Two illumination windows 9a and 9b are located near the observation window 10a, and two illumination windows 9c and 9d are located near the observation window 10b. Therefore, two observation windows 10a and 10b are arranged at substantially equal angles in the circumferential direction of the insertion portion 6.

The observation window 8 configures a first subject image acquisition portion that acquires an image from the forward direction included in the first region. The observation window 10a and the observation window 10b configure second subject image acquisition portions that acquire images from the lateral direction included in the second region different from the forward direction.

More specifically, the image from the forward direction included in the first region is a subject image of the first region including the forward direction of the insertion portion 6 substantially parallel to the longitudinal direction of the insertion portion 6. The images from the lateral direction included in the second region are subject images of the second region including the lateral direction of the insertion portion 6 in a direction intersecting (for example, substantially orthogonal to) the longitudinal direction of the insertion portion 6. The observation window 8 is a forward subject image acquisition portion that acquires the subject image of the first region including the forward direction of the insertion portion 6. The observation windows 10 are lateral subject image acquisition portions that acquire the subject images of the second region including the lateral direction of the insertion portion 6.

The observation window 8 as a subject image acquisition portion is arranged on the distal end portion 6a of the insertion portion 6 in a direction in which the insertion portion 6 is inserted. The observation window 10a and the observation window 10b as subject image acquisition portions are arranged on the side surface portion of the insertion portion 6, in the outer diameter direction of the insertion portion 6.

As shown in Fig. 11, a first image pickup unit 14a for lateral field of view is located in the distal end portion 6a, on a back side of the observation window 10a. A second image pickup unit 14b for lateral field of view is located in the distal end portion 6a, on a back side of the observation window 10b. An image pickup unit 14c for forward field of view is located in the distal end portion 6a, on a back side of the observation window 8 for forward field of view.

Each of the three image pickup units 14a, 14b, and 14c includes an image pickup device and is controlled by the processor 4. The image pickup unit 14c photoelectrically converts the subject image from the observation window 8. The image pickup unit 14a photoelectrically converts the subject image from the observation window 10a, and the image pickup unit 14b photoelectrically converts the subject image from the observation window 10b, respectively. The image pickup unit 14a and the image pickup unit 14b output respective image pickup signals to the electrically connected processor 4.

The processor 4 includes a control section 42C, a photometric section 41A, and an illumination control section 31C. The control section 42C of the processor 4 serves as an image generation section to generate three endoscopic images based on three image pickup signals from the three image pickup units 14a, 14b, and 14c and outputs the three endoscopic images to the display apparatus 5.

Fig. 12 is a diagram showing an example of a display screen of the endoscopic images displayed on the display apparatus 5.

Three endoscopic images are displayed on the display screen 5a of the display apparatus 5. A first region 51 is a region for displaying a first lateral observation image generated from the image pickup signal from the image pickup unit 14a. A second region 52 is a region for displaying a forward observation image generated from the image pickup signal from the image pickup unit 14c. A third region 53 is a region for displaying a second lateral observation image generated from the image pickup signal from the image pickup unit 14b.

As shown in Fig. 12, the three endoscopic images are lined up and displayed on the display screen 5a of the display apparatus 5 (that is, the processor 42 lines up and arranges the lateral images and the forward image adjacent to each other). The photometric section 41A of the processor 4 calculates the brightness of each of the three endoscopic images generated by the processor 4 and outputs the brightness to the control section 42C.

The processor 4 is an image processing section configured to generate image signals including the forward observation image and the two lateral observation images. The control section 42C controls the amount of light of the corresponding illumination light according to the brightness of each endoscopic image and performs gain adjustment of each image signal.

The display apparatus 5 configures a display section that receives the image signals from the processor 4 to display the endoscopic images including the forward observation image and the two lateral observation images such that the two lateral observation images are displayed next to (adjacent to) the forward observation image. Here, the processor 4 displays the two lateral observation images on the display apparatus 5 so as to sandwich the forward observation image. That is, the processor 4 generates the images in which the subject image of the forward field of view is arranged at the center, and the two subject images of the lateral field of view are lined up and arranged to sandwich the subject image of the forward field of view.

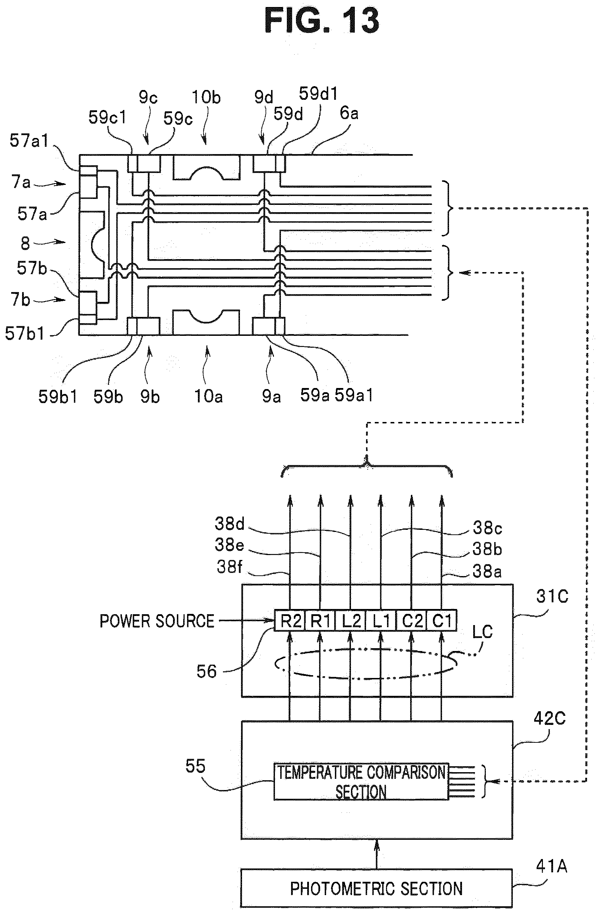

Fig. 13 is a diagram for describing drive control of six light-emitting devices respectively located on the six illumination windows 7a, 7b, 9a, 9b, 9c, and 9d located on the distal end portion 6a.

Light-emitting devices 57a and 57b are located on the illumination windows 7a and 7b for forward field of view, respectively, and the light-emitting device 57a and 57b configure a first illumination portion that emits first illumination light to a region including the forward direction as a first region inside of the subject. The light-emitting devices 57a and 57b are connected to the illumination control section 31C through signal lines 38a and 38b, respectively. Temperature sensors 57a1 and 57b1 are further provided near the light-emitting devices 57a and 57b, respectively. The six light-emitting devices are, for example, light-emitting diodes (LEDs).

Light-emitting devices 59a and 59b are located on the first illumination windows 9a and 9b for lateral field of view, respectively. The light-emitting devices 57a and 57b are connected to the illumination control section 31C through signal lines 38c and 38d, respectively. Temperature sensors 59a1 and 59b1 are further provided near the light-emitting devices 59a and 59b, respectively.

Light-emitting devices 59c and 59d are located on the second illumination windows 9c and 9d for lateral field of view, respectively. The light-emitting devices 59c and 59d are connected to the illumination control section 31C through signal lines 38e and 38f, respectively. Temperature sensors 59c1 and 59d1 are further provided near the light-emitting devices 59c and 59d, respectively.

The first illumination windows 9a and 9b for lateral field of view and the second illumination windows 9c and 9d for lateral field of view configure a second illumination portion that emits second illumination light to a region including the lateral direction as a second region at least partially different from the first direction.

The second region different from the first region denotes that optical axes in the respective regions are in different directions. A subject image in the first region and a subject image in the second region may partially overlap or may not overlap. Furthermore, an irradiation range of the first illumination light and an irradiation range of the second illumination light may partially overlap or may not overlap.

In this way, each illumination light for forward field of view and for lateral field of view is generated by light emission of the light-emitting device.

The control section 42C includes a temperature comparison section 55. The temperature comparison section 55 is a circuit configured to generate temperature data of each light-emitting device based on the output signal of each temperature sensor and compare whether the temperature data is equal to or greater than a predetermined value TH3. Therefore, the temperature comparison section 55 configures a signal detection section that detects signals indicating temperatures of the illumination windows 7a, 7b, and 9a to 9d. More specifically, the signals indicating the temperatures include first signals that are output signals of the temperature sensors 57a1 and 57b1 provided near the light-emitting devices 57a and 57b for forward field of view and second signals that are output signals of the temperature sensors 59a1, 59b1, 59c1, and 59d1 provided near the light-emitting devices 59a, 59b, 59c, and 59d for lateral field of view.

The illumination control section 31C includes an output limiter section 56. The output limiter section 56 is a circuit configured to limit a drive signal for causing each light-emitting device to emit light to a predetermined signal level for each light-emitting device. Here, the output limiter section 56 includes six limiter circuits corresponding to the six light-emitting devices 57a, 57b, 59a, 59b, 59c, and 59d. In Fig. 13, limiter circuits C1, C2, L1, L2, R1, and R2 are circuits for limiting the drive signals supplied to the light-emitting devices 57a, 57b, 59a, 59b, 59c, and 59d, respectively.

The temperature comparison section 55 is a processing section configured to generate temperature data of each temperature sensor from the output signals of the temperature sensors 57a1, 57b1, 59a1, 59b1, 59c1, and 59d1. For example, the temperature data of the temperature sensor 57a1 is calculated based on the output signal of the temperature sensor 57a1.

The temperature comparison section 55 further compares whether the temperature data of each temperature sensor is equal to or greater than the predetermined value TH3. If the temperature of each temperature sensor is equal to or greater than the predetermined value TH3, the temperature comparison section 55 outputs a limit control signal LC to the output limiter section 56 configured to limit the drive signal for the light-emitting device provided with the temperature sensor equal to or greater than the predetermined value TH3.

If the temperature of each temperature sensor is equal to or greater than the predetermined value TH3, the temperature comparison section 55 outputs the limit control signal LC to the output limiter section 56 to limit the drive signal for the light-emitting device near the temperature sensor equal to or greater than the predetermined value TH3 to prevent the temperature from becoming equal to or greater than a predetermined value. The predetermined value TH3 is, for example, 37°C.

For example, if the temperature data of the temperature sensor 59c1 provided near the light-emitting device 59c of the first field of view region becomes equal to or greater than the predetermined value TH3, an upper limit of only the drive signal for the light-emitting device 59c is lowered by a predetermined value.

In this way, the temperature comparison section 55 and the output limiter section 56 configure an illumination light amount control section configured to control the amount of light of at least one of the illumination windows 7a, 7b, and 9a to 9d based on the signals indicating the temperatures of the illumination windows for forward field of view and the signals indicating the temperatures of the illumination windows for lateral field of view. More specifically, the temperature comparison section 55 and the output limiter section 56 as the illumination light amount control section limit the drive signals of the light-emitting devices for forward field of view or the light-emitting devices for lateral field of view to control at least one of the amounts of light.

As a result, the signal level of the output signal for the light-emitting device equal to or greater than the predetermined value TH3 is lowered, and overheating of the distal end portion is suppressed.

Therefore, according to the present embodiment, the temperatures of two or more illumination windows with different illumination regions are individually checked in an endoscope that can observe two or more directions. The amounts of light for the two or more illumination windows are individually and independently controlled to prevent the temperatures from rising above a predetermined temperature. This can provide an endoscope system that can perform detailed illumination control in which the amounts of light of all illuminations do not change at the same time and that can prevent overheating of the distal end portion.

### (Fifth Embodiment)

In the endoscope system of the fourth embodiment, the temperature sensor detects the temperature of each light-emitting device, and the maximum output of the drive signal for each light-emitting device is limited according to the temperature of each light-emitting device. The temperature sensor is not used in an endoscope system of the present embodiment, and when a most recent integrated value of the drive signal exceeds a predetermined value based on a most recent change over time of the drive signal for each light-emitting device, a light adjustment level of an image, that is, brightness of an image, obtained by the light-emitting device in which the most recent integrated value of the drive signal exceeds the predetermined value is lowered by a predetermined value to lower a signal level of the drive signal as a result.

A configuration of the endoscope system of the present embodiment is substantially the same as the configuration of the endoscope system 1C of the fourth embodiment. The same reference signs are provided to the same constituent elements, and the description will not be repeated. Different components will be described.

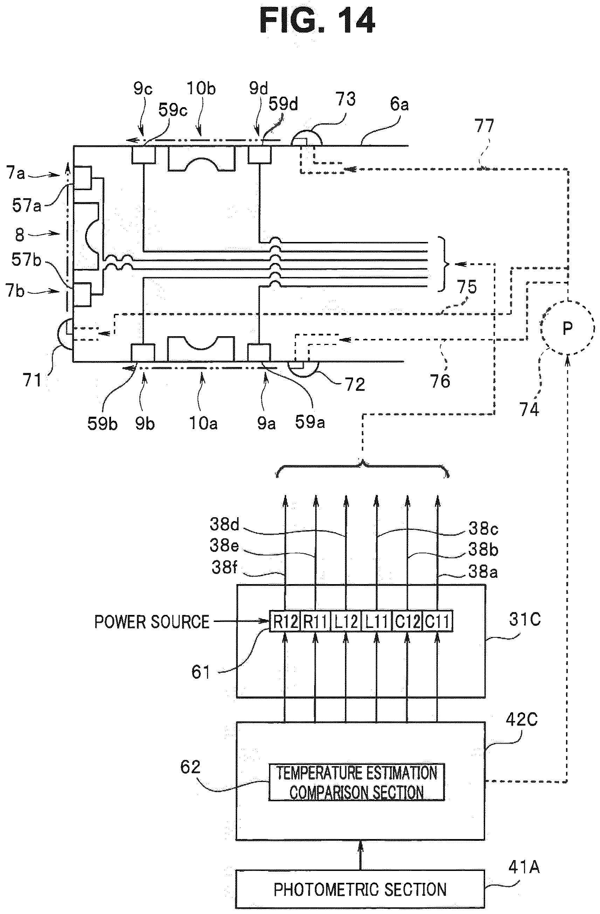

Fig. 14 is a diagram for describing drive control of the six light-emitting devices respectively located on the six illumination windows 7a, 7b, 9a, 9b, 9c, and 9d located on the distal end portion 6a. As shown in Fig. 14, the temperature sensor is not provided on the distal end portion 6a of the insertion portion 6.

The illumination control section 31C includes a light-emitting device drive section 61 configured to drive each of the light-emitting devices 57a, 57b, 59a, 59b, 59c, and 59d. The light-emitting device drive section 61 includes six drive circuits. Drive circuits C11, C12, L11, L12, R11, and R12 of the light-emitting device drive section 61 are circuits configured to drive the light-emitting devices 57a, 57b, 59a, 59b, 59c, and 59d, respectively.

The control section 42C includes a temperature estimation comparison section 62. The temperature estimation comparison section 62 is a processing section configured to calculate an integrated value of the size of the drive signal for each light-emitting device in a past predetermined period PT1, such as an integrated value of the size of a current value or a power value, estimate the temperature of each light-emitting device from the most recent integrated value, and compare whether the most recent integrated value is equal to or greater than a predetermined value TH4. Therefore, the temperature estimation comparison section 62 configures a signal detection section that estimates and detects the signals indicating the temperatures of the illumination windows 7a, 7b, and 9a to 9d. More specifically, the signals indicating the temperatures include a first signal that is a signal of the integrated value of the drive signal for the light-emitting device for forward field of view in the predetermined time period PT1 and a second signal that is a signal of the integrated value of the drive signal for the light-emitting device for lateral field of view in the predetermined time period PT1.

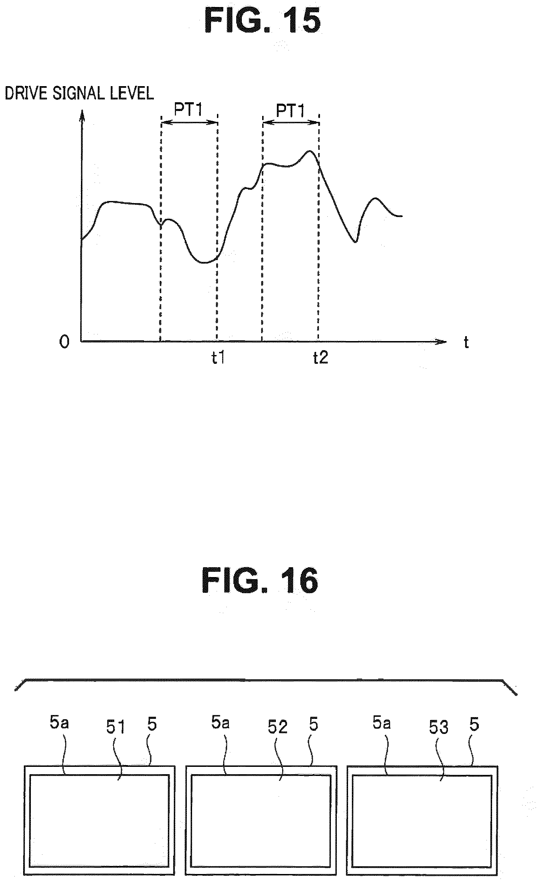

Fig. 15 is a graph showing a change in the drive signal level for the light-emitting device with a lapse of time period. As shown in Fig. 15, the drive signal level for the light-emitting device changes as indicated by a solid line. As described, the drive signal level for the light-emitting device is controlled by the control section 42C and changes based on the brightness of each of the two regions 22 and 23 of the endoscopic image 21 detected by the photometric section 41.

Comparing the time t1 and the time t2 in Fig. 15 for example, the drive signal level for the light-emitting device in the most recent past predetermined period PT1 at the time t2 is greater than the drive signal level for the light-emitting device in the most recent past predetermined period PT1 at the time t1. Therefore, the integrated value of the drive signal level for the light-emitting device in the past predetermined period PT1 is also greater at the time t2 than at the time t1.

When the integrated value of the most recent past drive signal level is large, the temperature of the distal end portion 6a may be rising. That is, when the integrated value of the drive signal level in the most recent predetermined period PT1 is equal to or greater than the predetermined value TH4, it is estimated that the temperature of the distal end portion 6a is about to exceed a predetermined temperature, such as 37 degrees, or is already exceeding the predetermined temperature.

Therefore, the control section 42C lowers, by a predetermined value D, the target value of the brightness of the endoscopic image obtained through the observation window corresponding to the light-emitting device in which the integrated value of the drive signal level is equal to or greater than the predetermined value TH4, and as a result, the drive signal level of the light-emitting device is lowered by the predetermined value DL.

Therefore, the control section 42C including the temperature estimation comparison section 62 serves as a signal detection section to detect the signals indicating the temperatures of the illumination windows for forward field of view and the signals indicating the temperatures of the illumination windows for lateral field of view and configures an illumination light amount control section configured to control the amount of light of at least one of the illumination windows 7a, 7b, and 9a to 9d based on the signals. More specifically, the control section 42C lowers the target value of the brightness of the subject image of the forward field of view or the subject image of the lateral field of view to control at least one of the amounts of light.

Therefore, an amount of heat generation of the light-emitting device in which the integrated value of the drive signal level is equal to or greater than the predetermined value TH4 is reduced, and a rise in the temperature of the distal end portion 6a can be suppressed.

In this way, the control section 42C lowers the target value of the brightness by the predetermined value D when the temperature of the light-emitting device of the distal end portion 6a becomes equal to or greater than the predetermined temperature. As a result, the drive signal level of the light-emitting device is lowered by the predetermined value DL, and a rise in the temperature of the distal end portion 6a can be suppressed.

Note that although the target value of the brightness is lowered by the predetermined value D based on whether the integrated value of the drive signal level for the light-emitting device in the most recent predetermined period PT1 is equal to or greater than the predetermined value TH4 in the example, the drive signal level may be lowered to reduce the brightness target value of the image in stages according to the integrated value of the drive signal level.

For example, when the integrated value of the drive signal level of the light-emitting device is between DC1 and DC2, the target value of the brightness may be reduced by 10%. When the integrated value of the drive signal level is between DC2 and DC3, the target value of the brightness may be reduced by 20%. When the integrated value of the drive signal level is equal to or greater than DC3, the target value of the brightness may be reduced by 30%.

In this case, when the integrated value of the drive signal level subsequently decreases, and the integrated value of the drive signal level becomes between DC2 and DC3, the target value of the brightness is increased to the level of 20% reduction. When the integrated value of the drive signal level becomes between DC1 and DC2, the target value of the brightness is increased to the level of 10% reduction. When the drive signal level becomes less than DC1, the target value of the brightness is not reduced.

Therefore, according to the present embodiment, the temperatures of two or more illumination windows with different illumination regions are individually estimated, and the amounts of light for the two or more illumination windows are individually and independently controlled to prevent the temperatures from rising above a predetermined temperature in an endoscope that can observe two or more directions. This can provide an endoscope system that can perform detailed illumination control in which the amounts of light of all illuminations do not change at the same time and that can prevent overheating of the distal end portion.

There is also an endoscope 2B in which cleaning nozzles for cleaning the observation windows are provided on the distal end portion 6a, and water feeding conduits are provided in the insertion portion 6. A fluid supply switch provided on an operation portion not shown is operated, and water for cleaning is discharged from the cleaning nozzles. When water is fed for cleaning, the temperature of the distal end portion 6a drops.

Therefore, if the fluid supply switch is operated to feed water for cleaning when the integrated value of the drive signal level in the most recent predetermined period PT1 is equal to or greater than the predetermined value TH4, that is, when the temperature of the light-emitting device becomes equal to or higher than a predetermined temperature, the target value of the brightness of the image may be raised while the water is fed.

As shown in Fig. 14, cleaning nozzles 71, 72, and 73 are located on the distal end portion 6a, near the respective observation windows. The nozzles 71, 72, and 73 are connected to a pump 74 provided on a light source apparatus or the like not shown, through water feeding conduits 75, 76, and 77, respectively. The pump 74 acts according to a pump drive signal from the control section 42C based on the operation portion (not shown) of the endoscope 2B. Water is discharged from the respective cleaning nozzles 71, 72, and 73 as indicated by two-dot chain lines.

Therefore, when the integrated value of the drive signal level for the light-emitting device in the most recent predetermined period PT1 is equal to or greater than the predetermined value TH4, the target value of the brightness of the image obtained through the observation window corresponding to the light-emitting device may be raised by a predetermined value D1, or the target value may be returned to the target value before the target value is lowered by the predetermined value D, while the water is fed. This is because a cooling effect of water feeding suppresses a rise in the temperature of the light-emitting device even if the target value of the brightness of the image is raised. That is, the control section 42C raises the target value of the brightness of the image when water is fed from the cleaning nozzle 71 or the like provided on the insertion portion 6.

Therefore, according to the present embodiment, the temperatures of two or more illumination windows with different illumination regions are individually checked in an endoscope that can observe two or more directions. The amounts of light for the two or more illumination windows are individually and independently controlled to prevent the temperatures from rising above a predetermined temperature. This can provide an endoscope system that can perform detailed illumination control in which the amounts of light of all illuminations do not change at the same time and that can prevent overheating of the distal end portion.

Note that although three observation images are displayed on the display screen of one display apparatus in the fourth and fifth embodiments, a plurality of display apparatuses 5 arranged adjacent to each other may be used to display the forward observation image and the lateral observation images on separate display screens 5 a.

Fig. 16 is a diagram showing a display system using three display apparatuses 5. As shown in Fig. 16, the first display region 51 is displayed on the display screen 5 a of the display apparatus 5 on the left side, and the second display region 52 is displayed on the display screen 5a of the display apparatus 5 at the center. The third display region 53 is displayed on the display screen 5a of the display apparatus 5 on the right side.

A mode of respectively displaying the forward observation image and the lateral observation images on the plurality of separate display screens 5a and a mode of displaying the forward observation image and the lateral observation images on one display screen 5a shown in Fig. 12 may be able to be switched by switch operation or the like.

Note that although the mechanism for realizing the function of illuminating and observing the lateral direction is embedded in the insertion portion 6 along with the mechanism for realizing the function of illuminating and observing the forward direction in each of the embodiments, the mechanism for realizing the function of illuminating and observing the lateral direction may be a separate body that can be attached to and detached from the insertion portion 6.

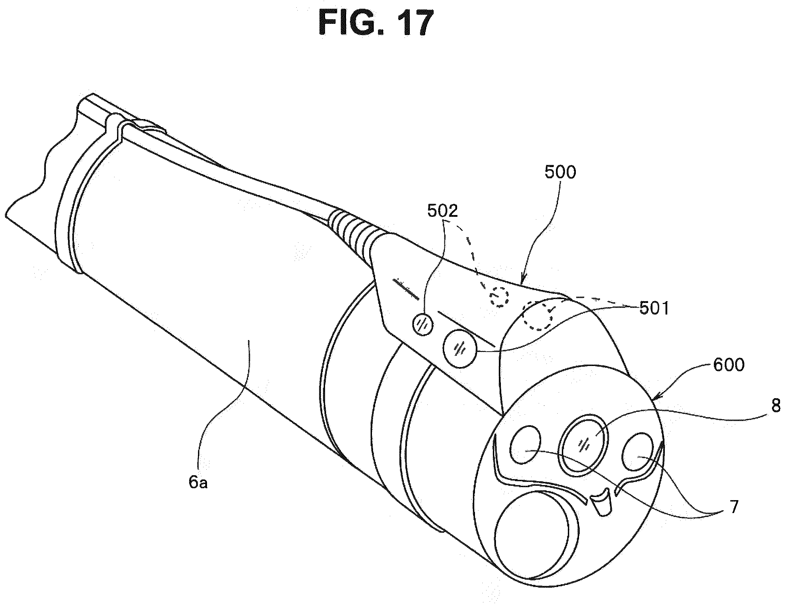

Fig. 17 is a perspective view of the distal end portion 6a of the insertion portion 6 in which a unit for lateral observation is attached. The distal end portion 6a of the insertion portion 6 includes a forward field of view unit 600. A lateral field of view unit 500 is attachable to and detachable from the forward field of view unit 600.

The lateral field of view unit 500 includes: two observation windows 501 for acquiring images in the left and right directions; and two illumination windows 502 for illuminating the left and right directions.

Each of the embodiments can also be applied to an endoscope system including the insertion portion 6 as shown in Fig. 16.

Note that in each of the embodiments and each of the modifications, the image generation section may combine the image of the forward field of view (forward observation image) and the images of the lateral field of view (lateral observation images) to display one combined image as an endoscopic image on the display screen 4a. The image generation section may display endoscopic images including a plurality of simply lined up images, such as two or three images, on the display screen 4a without combining the image for the forward field of view (forward observation image) and the images of the lateral field of view (lateral observation images).

The present invention is not limited to the embodiments, and various changes, modifications, and the like can be made without changing the scope of the present invention.

The present application is filed on the basis of claiming the benefit of priority from Japanese Patent Application No. 2014-153103, filed in Japan on July 28, 2014, the disclosed content of which is incorporated in the present specification and claims by reference.

## Claims

1. An endoscope system comprising:
an insertion portion inserted into a subject;
a subject image acquisition portion provided on the insertion portion and configured to acquire an image of the subject;
a first illumination portion provided on the insertion portion and configured to emit first illumination light to a first region of the subject;
a second illumination portion provided on the insertion portion and configured to emit second illumination light to a second region of the subject at least partially different from the first region;
a signal detection section configured to detect a first signal indicating a temperature of the first illumination portion and a second signal indicating a temperature of the second illumination portion; and
an illumination light amount control section configured to individually and independently control an amount of light of the first illumination light and an amount of light of the second illumination light based on the first signal and the second signal.

2. The endoscope system according to claim 1, wherein
the illumination light amount control section limits a maximum aperture value of an aperture for limiting the amounts of light of the first and second illumination lights to control at least one of the amounts of light.

3. The endoscope system according to claim 1, wherein
the signals indicating the temperatures are signals of amounts of opening of an aperture for limiting the amounts of light of the first and second illumination lights.

4. The endoscope system according to claim 1, wherein
the signals indicating the temperatures are signals of integrated values of amounts of opening of an aperture for limiting the amounts of light of the first and second illumination lights in a predetermined time period.

5. The endoscope system according to claim 4, wherein
the illumination light amount control section lowers a target value of brightness of the first subject image and the second subject image to control at least one of the amounts of light.

6. The endoscope system according to claim 1, wherein
the first illumination light is generated by light emission of a first light-emitting device,
the second illumination light is generated by light emission of a second light-emitting device, and
the signals indicating the temperatures include, as the first signal, an output signal of a first temperature sensor provided near the first light-emitting device and , as the second signal, an output signal of a second temperature sensor provided near the second light-emitting device.

7. The endoscope system according to claim 6, wherein
the illumination light amount control section limits a drive signal of the first light-emitting device or the second light-emitting device to control at least one of the amounts of light.

8. The endoscope system according to claim 1, wherein
the first illumination light is generated by light emission of a first light-emitting device,
the second illumination light is generated by light emission of a second light-emitting device, and
the signals indicating the temperatures include, as the first signal, a signal of a first integrated value of a drive signal for the first light-emitting device in a predetermined time period and, as the second signal, a signal of a second integrated value of a drive signal for the second light-emitting device in the predetermined time period.

9. The endoscope system according to claim 8, wherein
the illumination light amount control section lowers a target value of brightness of the first subject image and the second subject image to control at least one of the amounts of light.

10. The endoscope system according to claim 9, wherein
the illumination light amount control section raises the target value of the brightness when water is fed from a cleaning nozzle provided on the insertion portion.

11. The endoscope system according to claim 1, wherein
each of the first and second illumination portions includes a light guide, and
the first illumination light and the second illumination light are supplied through the light guide.

12. The endoscope system according to claim 1, wherein
the subject image acquisition portion includes: a first subject image acquisition portion configured to acquire a first subject image from the first region including an insertion portion forward direction substantially parallel to a longitudinal direction of the insertion portion; and
a second subject image acquisition portion configured to acquire a second subject image from the second region including an insertion portion lateral direction in a direction intersecting the longitudinal direction of the insertion portion.

13. The endoscope system according to claim 12, further comprising:
an image generation section configured to generate a first image based on the first subject image and a second image based on the second subject image; and
a display section configured to display the first and second images generated by the image generation section, wherein
the image generation section lines up and arranges the second image adjacent to the first image.

14. The endoscope system according to claim 12, wherein
the first subject image acquisition portion is provided on a distal end portion in the longitudinal direction of the insertion portion,
the second subject image acquisition portion is provided in a circumferential direction of the insertion portion, and
an image pickup section configured to photoelectrically convert the first subject image and the second subject image on one image pickup surface is electrically connected to an image generation section configured to generate a first image based on the first subject image and a second image based on the second subject image.

15. The endoscope system according to claim 14, wherein
the first subject image is substantially circular, and
the second subject image has a substantially annular shape surrounding at least part of surroundings of the first subject image.
